(19) Europäisches Patentamt
European Patent Office
Office européen des brevets

(11) EP 2 386 300 A1

(12) **EUROPEAN PATENT APPLICATION**

(43) Date of publication:
**16.11.2011 Bulletin 2011/46**

(51) Int Cl.:
*A61K 31/343* *(2006.01)*    *A61P 9/00* *(2006.01)*

(21) Application number: **10305504.2**

(22) Date of filing: **11.05.2010**

| | |
|---|---|
| (84) Designated Contracting States:<br>**AL AT BE BG CH CY CZ DE DK EE ES FI FR GB GR HR HU IE IS IT LI LT LU LV MC MK MT NL NO PL PT RO SE SI SK SM TR**<br>Designated Extension States:<br>**BA ME RS** | (72) Inventor: **The designation of the inventor has not yet been filed** |
| | (74) Representative: **Bouron, Estelle et al**<br>**Sanofi-Aventis**<br>**Département Brevets**<br>**174, avenue de France**<br>**75013 Paris (FR)** |
| (71) Applicant: **SANOFI**<br>**75013 Paris (FR)** | |

(54) **Use of dronedarone for the preparation of a medicament for use in the prevention of cardiovascular hospitalization or of mortality in patients having a first recurrence of atrial fibrillation or atrial flutter**

(57)    Use of dronedarone or pharmaceutically acceptable salts thereof, for the preparation of a medicament for use in the prevention of cardiovascular hospitalization or of mortality in patients having a first recurrence of atrial fibrillation or atrial flutter.

EP 2 386 300 A1

**Description**

The present invention relates to the use of dronedarone or pharmaceutically acceptable salts thereof, for the preparation of a medicament for use in the prevention of cardiovascular hospitalization and/or of mortality in patients having a first recurrence of atrial fibrillation (AF) or atrial flutter (AFL).

2-n-Butyl-3-[4-(3-di-n-butylaminopropoxy)benzoyl]-5-methylsulphonamidobenzofuran, or dronedarone, and pharmaceutically acceptable salts thereof are described in European Patent EP 0 471 609 B1.

Dronedarone blocks potassium, sodium and calcium channels and also has anti-adrenergic properties.

Dronedarone is an anti-arrhythmic that is effective in maintaining sinus rhythm in patients presenting atrial fibrillation or atrial flutter.

The applicant has clinically proven that dronedarone significantly reduces cardiovascular hospitalizations and/or mortality in patients having a history of atrial fibrillation or of atrial flutter.

Atrial fibrillation (AF) affects about 2.3 million people in North America and 4.5 million people in the European Union and is emerging as a growing public health concern because of the aging of the population.

AF is a condition in which the upper chambers of the heart beat in an uncoordinated and disorganized fashion, resulting in a very irregular and fast rhythm (i.e., an irregularly, irregular heartbeat). When blood is not completely pumped out of the heart's chambers, it can pool and clot. If a blood clot forms in the atria, exits the heart and blocks an artery in the brain, a stroke results. Consequently, about 15 percent of strokes result from AF.

AF is increasingly frequent with advancing age and is often caused by age-related changes in the heart, physical or psychological stress, agents that stimulate the heart, such as caffeine, or as a result of cardiovascular disease. The number is expected to double in the next 20 years. Without appropriate management, AF can lead to serious complications, such as stroke and congestive heart failure.

It is known that atrial fibrillation itself can cause changes in the electrical parameters of the heart known as electrical remodelling and in the structure of the cardiac chambers known as structural remodelling which tend to decrease the chances of the patient to get back into normal sinus rhythm. This vicious circle whereby "atrial fibrillation begets atrial fibrillation" has been well documented since the 1990s (Wijffels MC, Kirchhof CJ, Dorland R, Allessie MA.Atrial fibrillation begets atrial fibrillation. A study in awake chronically instrumented goats.Circulation. 1995 Oct 1;92(7): 1954-68.). It explains why when patients have been in atrial fibrillation for a long time they develop permanent atrial fibrillation with little or no chance to recover from this arrhythmia which becomes chronic.

Generally speaking, when antiarrhytmic drugs have been evaluated for their efficacy, a recurrence of atrial fibrillation or atrial flutter for example may be regarded as a therapeutic failure, leading to the discontinuation of treatment.

The applicant has now surprisingly found and clinically proven that dronedarone or a pharmaceutically acceptable salt thereof preserves its beneficial effect even after the first recurrence of AF or AFL and consequently continuation of dronedarone administration after first recurrence of atrial fibrillation or atrial flutter may be clinically advantageous for patients.

A subject of the present invention is therefore the use of dronedarone or a pharmaceutically acceptable salt thereof, for the preparation of a medicament, for use in the prevention of cardiovascular hospitalizations and/or of mortality in patient having a first recurrence of atrial fibrillation or atrial flutter.

Said prevention of cardiovascular hospitalizations and/or of mortality is provided to patients having a history of atrial fibrillation or atrial flutter.

A subject of the present invention is therefore the use of dronedarone or a pharmaceutically acceptable salt thereof, for the preparation of a medicament for use in the prevention of cardiovascular hospitalizations and/or of mortality notably in patients having a history of atrial fibrillation or atrial flutter and a first recurrence of atrial fibrillation or atrial flutter.

Mention may in particular be made of cardiovascular mortality.

More specifically, a subject of the present invention is the use of dronedarone or a pharmaceutically acceptable salt thereof, for the preparation of a medicament for use in the prevention of approximately 24% of cardiovascular hospitalizations and/or of mortality in patients having a first recurrence of atrial fibrillation or atrial flutter.

A subject of the present invention is also the use of dronedarone or a pharmaceutically acceptable salt thereof, in patients having a first recurrence of atrial fibrillation or atrial flutter, for the preparation of a medicament for use in the prevention:

- of cardiovascular hospitalizations, and more particularly of approximately 23% of cardiovascular hospitalizations, and/or
- of mortality, particularly of approximately 44% of mortality, and/or
- of cardiovascular mortality, and more particularly of approximately 47% of cardiovascular mortality, and/or

Mention may be made that "use of dronedarone for the preparation of a medicament for use in the treatment of" may be understood as "dronedarone for the treatment of ".

In terms of clinical study, the prevention of "cardiovascular hospitalizations or of mortality" or of "cardiovascular hospitalizations or of cardiovascular mortality" constitute what are referred to as composite criteria or a combined endpoint.

Of course, it may be understood that "prevention of cardiovascular hospitalization and/or mortality" results in the reduction of the risk of cardiovascular hospitalization and or mortality or in the reduction of the need of cardiovascular hospitalization and or mortality.

All the percentages given above correspond to average values.

Among the pharmaceutically acceptable salts of dronedarone, mention may be made of the hydrochloride.

The term "cardiovascular hospitalization" means a hospitalization which is caused by at least one of the following pathologies (Hohnloser et al., Journal of cardiovascular electrophysiology, January 2008, vol. 19, No. 1, pages 69-73):

- relating to atherosclerosis,
- myocardial infarction or unstable angina pectoris,
- stable angina pectoris or atypical thoracic pain,
- syncope,
- transient ischemic event or cerebral stroke (except intracranial haemorrhage),
- atrial fibrillation and other supraventricular rhythm disorders,
- non-fatal cardiac arrest,
- ventricular arrhythmia,
- cardiovascular surgery, except heart transplant,
- heart transplant,
- implantation of a cardiac stimulator (pacemaker), of an implantable defibrillator ("ICD") or of another cardiac device,
- percutaneous coronary, cerebrovascular or peripheral intervention,
- variations in arterial pressure (hypotension, hypertension, except syncope),
- cardiovascular infection,
- major bleeding/haemorrhage (requiring two or more blood cell pellets or any intracranial haemorrhage),
- pulmonary embolism or deep vein thrombosis,
- worsening of congestive heart failure including acute pulmonary oedema or dyspnoea from cardiac causes.

Consequently, the prevention of cardiovascular hospitalization may be understood as the prevention of cardiovascular hospitalization for at least one of the above mentioned pathologies.

The term "mortality" or "death" are equivalent and cover mortality due to any cause, whether cardiovascular or non-cardiovascular or unknown.

The term "cardiovascular mortality" covers, in the context of the invention, mortality due to any cardiovascular causes (any death except those due to a non-cardiovascular cause), in particular death from an arrhythmic cause, also called arrhythmic death, and more particularly, sudden death from cardiovascular causes, also called sudden death or sudden cardiac death.

Cardiovascular mortality may be due for example to :

- Aortic dissection/aneurysm
- Cardiac tamponade
- Cardiogenic shock
- congestive heart failure
- Death during a cardiovascular transcutaneous interventional procedure or cardiovascular surgical intervention
- Hemorrhage (except cardiac tamponade)
- Myocardial infarction or unstable angina (including complications of myocardial infarction, except arrhythmias)
- Pulmonary or peripheral embolism
- Stroke
- Sudden cardiac death (eg, unwitnessed death or documented asystole)
- Ventricular arrhythmia, subclassified as torsades de pointes, ventricular extrasystole, ventricular fibrillation, ventricular tachycardia (non-sustained and sustained ventricular tachycardia), or other ventricular arrhythmia
- Unknown cause

The term "sudden death" or "sudden cardiac death" refers, in general, to death occurring within the hour or less than one hour after the appearance of new symptoms or unexpected death without warning.

In an embodiment, the subject of the instant invention is the use of dronedarone or one of its pharmaceutically acceptable salts for the preparation of a medicament for use in the prevention of cardiovascular hospitalization and/or of mortality notably in patients with a history of atrial fibrillation or atrial flutter and a first recurrence of atrial fibrillation or atrial flutter, said patient having at least one of the following characteristics:

- hypercholesterolemia,
- type 2 diabete,
- type 1 diabete,
- chronic renal failure,
- heart failure defined as NYHA class III or by a reduced left ventricular ejection fraction below 0.35.

NYHA means New York Heart Association.

According to one embodiement, patients may also have a history of atrial fibrillation or atrial flutter.

It will also be specified that the expression " patients having a history of atrial fibrillation or atrial flutter", "patients with a history of or a current atrial fibrillation or flutter" or "patients with a recent history of or a current atrial fibrillation or flutter" or "patients with paroxysmal or persistent atrial fibrillation or flutter" or "patients with a history of, or a current paroxysmal or persistent atrial fibrillation or flutter" or "patients with a recent history of, or a current paroxysmal or persistent atrial fibrillation or flutter" or "patients with paroxysmal or intermittent atrial fibrillation or atrial flutter and a recent episode of atrial fibrillation or atrial flutter, who are in sinus rhythm or who will be cardioverted" or "patients with paroxysmal or persistent atrial fibrillation or atrial flutter and a recent episode of atrial fibrillation or atrial flutter, who are in sinus rhythm or who will be cardioverted" means a patient who, in the past, has presented one or more episodes of atrial fibrillation or flutter and/or who is suffering from atrial fibrillation or atrial flutter at the time the dronedarone or a pharmaceutically acceptable salt thereof is used. More particularly, this expression means patients with documentation of having been in both atrial fibrillation or flutter and sinus rhythm within the last 6 months preceding the start of treatment. Patients could be either in sinus rhythm, or in atrial fibrillation or flutter at the time the dronedarone or a pharmaceutically acceptable salt thereof is initiated.

It will also be specified that the terms "persistent" and intermittent" are equivalent.

Among the patients with a recent history of, or a current atrial fibrillation or atrial flutter, mention may be made of patients with a history of, or a current, non permanent atrial fibrillation or flutter and in particular adult clinically stable patients with a history of , or current non-permanent AF.

Among the patients, notably patients having a history of atrial fibrillation or atrial flutter, mention may also be made of patients also exhibiting at least one of the following risk factors:

- age notably equal to or above 70, or even above 75,
- hypertension,
- diabetes,
- history of cerebral stroke or of systemic embolism, i.e. prior cerebrovascular accident,
- left atrial diameter greater than or equal to 50 mm measured for example by echocardiography,
- left ventricular ejection fraction less than 40%, measured for example by two-dimensional echography,

The efficacy of dronedarone to reduce cardiovascular hospitalization or death after first recurrence of atrial fibrillation or atrial flutter is now clinically proven in patients with the above mentioned associated risk factors.

The above mentioned risks factors may be defined as cardiovascular risk factors which are associated to atrial fibrillation.

Among the patients, notably patients having a history of atrial fibrillation or atrial flutter, mention may also be made of patients also exhibiting additional risk factors, i.e. at least one of the following pathologies:

- hypertension,
- underlying structural heart disease,
- tachycardia,
- coronary disease,
- non-rheumatic heart valve disease,
- dilated cardiomyopathy of ischemic origin,
- ablation of atrial fibrillation or flutter, for example catheter ablation or endomyocardial ablation,
- supraventricular tachycardia other than atrial fibrillation or flutter,
- history of heart valve surgery,
- non-ischemic dilated cardiomyopathy,
- hypertrophic cardiomyopathy,
- rheumatic valve disease,
- sustained ventricular tachycardia,
- congenital cardiopathy,
- ablation, for example catheter ablation, for tachycardia other than for atrial fibrillation or flutter,
- ventricular fibrillation,

and/or at least one cardiac device chosen from:

- a cardiac stimulator,
- an implantable defibrillator ("ICD").

For their therapeutic use, dronedarone and pharmaceutically acceptable salts thereof are generally introduced into pharmaceutical compositions.

These pharmaceutical compositions contain an effective dose of dronedarone or of a pharmaceutically acceptable salt thereof, and also at least one pharmaceutically acceptable excipient.

Said pharmaceutical composition may be given once or twice a day with food.

The dose of dronedarone administered per day, orally, may reach 800 mg, taken in one or more intakes, for example one or two.

More specifically, the dose of dronedarone administered may be taken with food.

More specifically, the dose of dronedarone administered per day, orally, may reach 800 mg, taken in two intakes with a meal.

The dose of dronedarone administered per day, orally may be taken at a rate of twice a day with a meal for example with the morning and the evening meal.

More specifically, the two intakes may comprise same quantity of dronedarone.

There may be specific cases where higher or lower dosages are appropriate; such dosages do not depart from the context of the invention. According to the usual practice, the dosage appropriate for each patient is determined by the physician according to the method of administration, the weight, the pathology, the body surface, the cardiac output and the response of said patient.

Said excipients are chosen according to the pharmaceutical form and the method of administration desired, from the usual excipients which are known to those skilled in the art.

In said pharmaceutical compositions for oral, sublingual, subcutaneous, intramuscular, intravenous, topical, local, intratracheal, intranasal, transdermal or rectal administration, dronedarone, or the salt thereof, can be administered in unit administration form, as a mixture with conventional pharmaceutical excipients, to animals and to humans in the cases mentioned above.

The suitable unit administration forms comprise forms for oral administration, such as tablets, soft or hard gel capsules, powders, granules and oral solutions or suspensions, sublingual, buccal, intratracheal, intraocular or intranasal administration forms, forms for administration by inhalation, topical, transdermal, subcutaneous, intramuscular or intravenous administration forms, rectal administration forms, and implants. For topical application, dronedarone and pharmaceutically acceptable salts thereof can be used in creams, gels, ointments or lotions.

By way of example, a unit administration form of dronedarone or a pharmaceutically acceptable salt thereof, in tablet form, may correspond to one of the following examples:

| Ingredients | mg | % |
|---|---|---|
| Dronedarone hydrochloride (corresponding to 400 mg of base) | 426 | 65.5 |
| Methylhydroxypropylcellulose | 21.1 | 3.25 |
| Lactose monohydrate | 46.55 | 7.2 |
| Maize starch | 45.5 | 7 |
| Polyvinylpyrrolidone | 65 | 10 |
| Poloxamer 407 | 40 | 6.15 |
| Anhydrous colloidal silica | 2.6 | 0.4 |
| Magnesium stearate | 3.25 | 0.5 |
|  | 650 | 100 |
| Dronedarone hydrochloride (corresponding to 400 mg of base) | 426 | 65.5 |
| Microcrystalline cellulose | 65 | 10 |
| Anhydrous colloidal silica | 2.6 | 0.4 |
| Anhydrous lactose | 42.65 | 6.6 |
| Polyvinylpyrrolidone | 13 | 2 |

(continued)

| Ingredients | mg | % |
|---|---|---|
| Poloxamer 407 | 40 | 6.15 |
| Macrogol 6000 | 57.5 | 8.85 |
| Magnesium stearate | 3.25 | 0.5 |
|  | 650 | 100 |

| Ingredients | mg |
|---|---|
| Dronedarone hydrochloride (corresponding to 400 mg of base) | 426 |
| Microcrystalline cellulose | 26 |
| Maize starch | 45.5 |
| Polyvinylpyrrolidone | 65 |
| Poloxamer 407 | 40 |
| Anhydrous colloidal silica | 3.25 |
| Magnesium stearate | 3.25 |
| Lactose monohydrate | 41.65 |
|  | 650 |
| Dronedarone hydrochloride (corresponding to 400 mg of base) | 213 |
| Microcrystalline cellulose | 13 |
| Maize starch | 22.75 |
| Polyvinylpyrrolidone | 32.5 |
| Poloxamer 407 | 20 |
| Anhydrous colloidal silica | 1.3 |
| Magnesium stearate | 1.625 |
| Lactose monohydrate | 20.825 |
|  | 650 |

According to another of its aspects, the present invention also relates to a method for treating the pathologies indicated above, which comprises the administration, to a patient having a first recurrence of atrial fibrillation or atrial flutter, of an effective dose of dronedarone or a pharmaceutically acceptable salt thereof.

One method of the invention includes decreasing the risk of mortality, cardiac hospitalizations, or the combination thereof in a patient having a first recurrence of atrial fibrillation or atrial flutter, said method comprising administering to said patient an effective amount of dronedarone or a pharmaceutically acceptable salt thereof, with food.

In one embodiment, the mortality is a cardiovascular mortality.

In some embodiments of the above methods according to the invention, the patient has a history of or current atrial fibrillation or flutter.

The present invention is illustrated by the data hereinafter with reference to the attached drawings in which:

Figure 1 represents a Kaplan Meier curve with the cumulative rate of hospitalization or of death from any cause over a period of 30 months;

Figure 2 represents a Kaplan Meier curve with the cumulative rate of hospitalization or of cardiovascular death over a period of 30 months;

Figure 3 represents the summary of nights of hospitalization for cardiovascular reasons among patients with at least one cardiovascular hospitalization.

The efficacy, relative to a placebo, of dronedarone and of pharmaceutically acceptable salts thereof, in the prevention of cardiovascular hospitalizations or of mortality was demonstrated, by means of dronedarone hydrochloride, in a prospective, multinational, multicentre, double-blind clinical study with random distribution in two groups of treatment (group treated with dronedarone hydrochloride and group treated with a placebo) of patients having a history of atrial fibrillation or atrial flutter.

More particularly, the effects of dronedarone compared with placebo on cardiovascular (CV) end points were evaluated by restarting the clock at the time of first atrial fibrillation or atrial flutter recurrence detected in a scheduled ECG or requiring cardioversion.

**I. Patient selection**

The patients had to have a history of atrial fibrillation or flutter and/or could be in normal sinus rhythm or in atrial fibrillation or flutter at inclusion.

The patient recruitment was carried out by taking into account the following inclusion criteria:

<u>Inclusion criteria:</u>

1) One of the following risk factors had to be present:

- age equal to or greater than 70 years,
- hypertension (taking antihypertensives of at least two different classes),
- diabetes,
- history of cerebral stroke (transient ischemic event or completed cerebral stroke) or of systemic embolism,
- left atrial diameter greater than or equal to 50 mm measured by echocardiography,
- left ventricular ejection fraction less than 40%, measured by two-dimensional echography;

or

- age equal to or above 70, or even above 75, possibly combined with at least one of the risk factors below:

    o hypertension (taking antihypertensives of at least two different classes),
    o diabetes,
    o history of cerebral stroke (transient ischemic event or completed cerebral stroke) or of systemic embolism,
    o left atrial diameter greater than or equal to 50 mm measured by echocardiography,
    o left ventricular ejection fraction less than 40%, measured by two-dimensional echography;

2) availability of an electrocardiogram carried out during the past 6 months in order to document the presence or the history of atrial fibrillation or flutter;

3) availability of an electrocardiogram carried out during the past 6 months in order to document the presence or absence of normal sinus rhythm.

<u>Exclusion criteria:</u>

<u>General criteria:</u>

- Refusal or inability to give informed consent to participate in the study.
- Any non cardiovascular illness or disorder that could preclude participation or severely limit survival including cancer with metastasis and organ transplantation requiring immune suppression
- Pregnant women (pregnancy test must be negative) or women or childbearing potential not on adequate birth control: only women with a highly effective method of contraception [oral] contraception or intra-uterine device (IUD) or sterile can be randomize.

- Breastfeeding women
- Previous (2 preceding months) or current participation in another trial with an investigational drug (under development) or with an investigational device.
- Previous participation in this trial.

Criteria Related to a cardiac condition:

- Patients in permanent atrial fibrillation
- Patients in unstable hemodynamic condition such as acute pulmonary edema within 12 hours prior to start of study medication; cardiogenic shock; treatment with IV pressor agents; patients on respirator; congestive heart failure of stage NYHA IV within the last 4 weeks; uncorrected, hemodynamically significant primary obstructive valvular disease; hemodynamically significant obstructive cardomyopathy; a cardiac operation or revascularization procedure within 4 weeks preceding randomization
- Planned major non-cardiac or cardiac surgery or procedures including surgery for valvular heart disease, coronary artery bypass graft (CABG), percutaneous coronary intervention (PCI), or on urgent cardiac transplantation list
- Acute myocarditis or constrictive pericarditis
- Bradycardia < 50 bpm and/or PR-interval $\geq$ 0.28 sec on the last 12-lead ECG.
- Significant sinus node disease (documented pause of 3 seconds or more) or 2nd or 3rd degree atrioventricular block (AV-block) unless treated with a pacemaker.

Criteria Related to Concomitant Medications:

Need of a concomitant medication that is prohibited in this trial, including the requirement for Vaughan Williams Class I and III anti-arrhythmic drugs, that would preclude the use of study drug during the planned study period, i.e. patients have to stop others antiarrhythmics such as Vaughan Williams Class I and III anti-arrhythmic drugs, for example amiodarone, flecainide, propafenone, quinidine, disopyramide, dofetilide, solatol.

Criteria Related to Laboratory Abnormalities:

- Plasma potassium < 3.5 mmol/I (as anti-arrhythmic drugs can be arrhythmogenic in patients with hypokalemia, this must be corrected prior to randomization.
- A calculated GFR at baseline < 10 ml/min using the Cockroft Gault formula (GFR [ml/min] = (140 - AGE [years] * WEIGHT [kilograms] * CONSTANT / CREATININE [$\mu$mol/L], where CONSTANT is 1 for men and 0.85 for women )[i]

Furthermore, the concomitant use of grapefruit juice and all potent inhibitors of CYP3A4 such as ketoconazole were prohibited.

**II. Duration and treatment**

Treatment was initiated using tablets containing either the placebo or an amount of dronedarone hydrochloride corresponding to 400 mg of dronedarone at a rate of twice a day with the morning and evening meal and more specifically at a rate of one tablet in the morning during or shortly after breakfast and one tablet in the evening during or shortly after dinner.

The anticipated duration of the treatment was variable according to the time at which each patient was included in the study, and could range from a minimum of 12 months for the last patient included up to a maximum corresponding to the entire duration of the study (12 months + duration of inclusion), i.e. approximately 30 months for the first patients included.

**III. Results**

The results obtained in this trial were analysed by the Kaplan Meier method for the figures, and the relative risk (RR) was estimated using Cox's proportional-effect regression model.

The relative risk (RR) is the ratio of the rates of occurrence of a hospitalization or of a death among the patients on dronedarone, relative to the patients on placebo.

The percentage reduction x of a given event (hospitalization, death, cardiovascular death, etc.) is calculated in the following way:

$$x = 1 - \text{relative risk.}$$

Baseline characteristics were comparable between patients with or without a first recurrence of AF (Table 1). Among patients who had a first recurrence, baseline characteristics were comparable between the dronedarone and placebo groups. Mean age was slightly lower, and there was a lower proportion of females in the group of patients with a first recurrence of atrial fibrillation or atrial flutter than in the group without a first recurrence.

**Table 1:**

| | Patients With a First AF/AFL Recurrence (n=1466) | | Patients Without a First AF/AFL Recurrence (n = 2007) | |
|---|---|---|---|---|
| | Overall | P Value[1] | Overall | P Value[1] |
| Mean age, y ($\pm$ SD) | 71 1 (9.0) | 0.0012 | 72.1(8.7) | 0.0076 |
| Female n(%) | 651 (44.4) | 0 1706 | 1044 (52 0) | 00042 |
| Hypercholesterolemia n (%) | 659 (45.0) | 0.9560 | 893 (44.5) | 0.4445 |
| Type 2 diabetes, n (%) | 248 (16.9) | 0 9443 | 356 (17.7) | 0.1982 |
| Type 1 diabetes n (%) | 41 (2 8) | 0 5271 | 55 (2 7) | 0.5871 |
| Chronic renal failure n (%) | 60 (4.1) | 1.0000 | 64 (3.2) | 0.8990 |
| Heart failure n (%) | 439 (29.9) | 0.3915 | 614 (30 6) | 0.7342 |
| NYHA class III | 61 (13.9) | 0 1260 | 82 (13.4) | 0 8127 |
| LVEF 35% | 48 (3.8) | 0.0791 | 63 (3.2) | 0.4420 |

- Dronedarone treatment was associated with a reduction of first AF recurrence compared with placebo (dronedarone, n = 673 [29.3%]; placebo, n = 793 [34.1%]):

- The risk for CV hospitalization (or all-cause mortality) in the placebo group after 1 year was 36.9% (5.1 %) in patients after their first AF recurrence compared with 29.6% (2.8%) in the entire ATHENA population.

- Overall, after a first AF recurrence, the primary composite end point occurred in 205 (30.5%) patients receiving dronedarone and 306 (38.6%) patients receiving placebo.

III.1. Results relating to cardiovascular hospitalizations or to mortality (principal judgement criterion)

Among the 1466 patients included in the study and who had a first recurrence of atrial fibrillation or atrial flutter, 673 were part of the group treated with dronedarone hydrochloride.

306 events were recorded in the placebo group, against 205 in the group treated with dronedarone hydrochloride.

The calculated relative risk is 0.76 with a p = 0.0025, i.e. a reduction in cardiovascular hospitalizations or deaths of 24% on dronedarone hydrochloride.

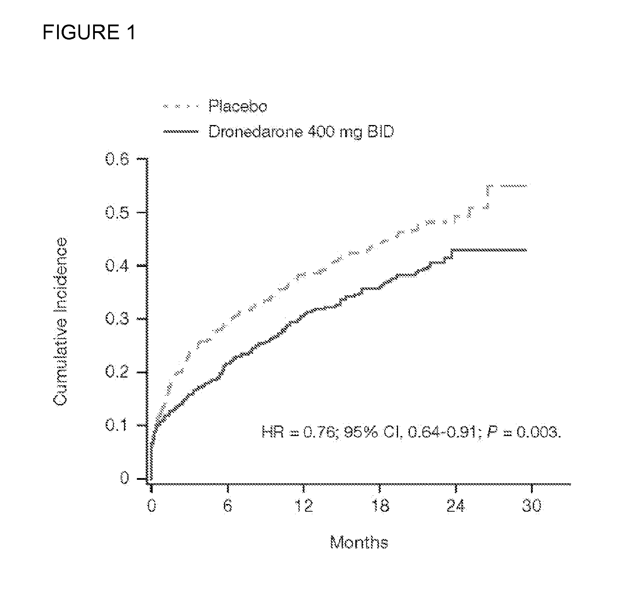

Figure 1, which reproduces the results obtained, shows a clear separation of the two cumulative curves very soon after the beginning of the treatment, this separation persisting over time throughout the duration of the study.

111.2. Results relating to cardiovascular hospitalizations

Among the 1466 patients included in the study and who had a first recurrence of atrial fibrillation or atrial flutter, 673 were part of the group treated with dronedarone hydrochloride.

288 events were recorded in the placebo group, against 195 in the group treated with dronedarone hydrochloride.

The calculated relative risk is 0.77 with a p = 0.0048, i.e. a reduction in cardiovascular hospitalizations of 23% on dronedarone hydrochloride.

15.8% and 21.2% of patients receiving dronedarone and placebo, respectively, were hospitalized for AF; 3.6% and 5.0% of dronedarone and placebo patients, respectively, were hospitalized for worsening heart failure (including pulmonary edema or dyspnea of cardiac origin).

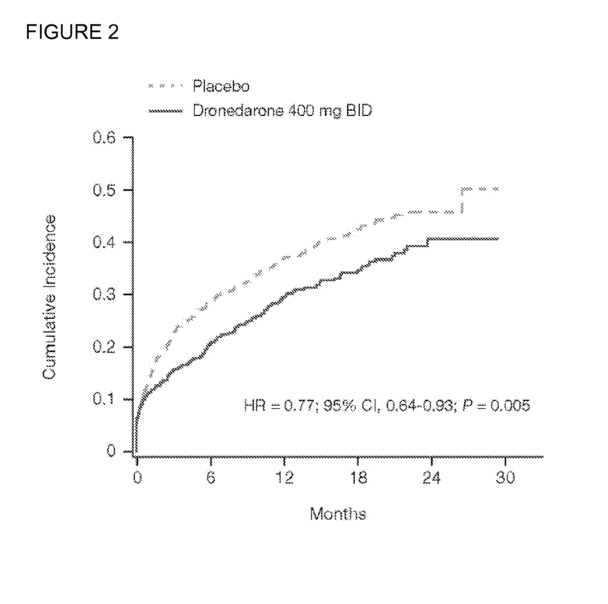

Figure 2, which reproduces the results obtained, shows a clear separation of the two cumulative curves very soon after the beginning of the treatment, this separation persisting over time throughout the duration of the study.

III.3. Results relating to time spent in hospital

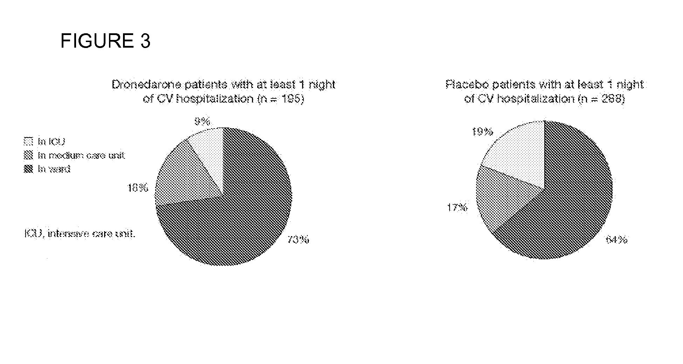

Among all patients with a first recurrence of atrial fibrillation or atrial flutter (1466), the total time spent in hospital was lower in the dronedarone group than in the placebo group (1273 nights and 1789 nights, respectively; P < 0.01). The percentage of hospitalization time spent in intensive care units (Figure 3) was lower in the dronedarone group than in the placebo group ($P$ = 0.01).

III.4. Results relating to mortality from any cause

Among the 1466 patients included in the study and who had a first recurrence of atrial fibrillation or atrial flutter, 673 were part of the group treated with dronedarone hydrochloride.

51 deaths were recorded in the placebo group, against 23 in the group treated with dronedarone hydrochloride.

The calculated relative risk is 0.56 with a p = 0.018, i.e. a reduction of death of 44% on dronedarone hydrochloride.

III.5. Results relating to cardiovascular mortality

Among the 1466 patients included in the study and who had a first recurrence of atrial fibrillation or atrial flutter, 673 were part of the group treated with dronedarone hydrochloride.

35 cardiovascular deaths were recorded in the placebo group, against 15 in the group treated with dronedarone hydrochloride.

The calculated relative risk is 0.53 with a p = 0.0342, i.e. a reduction in cardiovascular mortality of 47% on dronedarone hydrochloride.

**Claims**

1. Use of dronedarone or a pharmaceutically acceptable salt thereof, for the preparation of a medicament for use in the prevention of mortality and/or of cardiovascular hospitalizations in patients having a first recurrence of atrial fibrillation or atrial flutter.

2. Use according to Claim 1, for the preparation of a medicament for use in the prevention of cardiovascular hospitalizations.

3. Use according to Claim 1, for the preparation of a medicament for use in the prevention of mortality.

4. Use according to either of Claims 1 and 3, **characterized in that** the mortality is a cardiovascular mortality.

5. Use according to either of Claims 1, 2, 3 and 4, wherein said patients have a history of, or a current atrial fibrillation or atrial flutter.

6. Use according to either of Claims 1, 2, 3 and 4, wherein said patients have paroxysmal or persistent atrial fibrillation or atrial flutter

7. Use according to either of Claims 6, wherein said patients have a recent episode of atrial fibrillation or atrial flutter, who are in sinus rhythm or who will be cardioverted.

8. Use according to either of Claims 1, 2, 3 and 4, **characterized in that** patients have a history of or a current, non permanent atrial fibrillation or atrial flutter.

9. Use according to anyone of the preceding claims, **characterized in that** said patients have at least one of the following characteristics:

   - hypercholesterolemia,
   - type 2 diabete,
   - type 1 diabete,
   - chronic renal failure,
   - heart failure defined as NYHA class III or by a reduced left ventricular ejection fraction below 0.35.

10. Use according to anyone of the preceding claims, **characterized in that** the patients also exhibit associated risk factors.

11. Use according to anyone of the preceding claims, **characterized in that** the patients also exhibit at least one of the following risk factors:

   - age,
   - diabetes,
   - history of cerebral stroke or of systemic embolism,
   - left atrial diameter greater than or equal to 50 mm measured by echocardiography,
   - left ventricular ejection fraction less than 40%, measured by two-dimensional echography.

12. Use according to anyone of the preceding claims, **characterized in that** the patients also exhibit additional risk factors, i.e. at least one of the following pathologies:

   - hypertension,
   - underlying structural heart disease,
   - tachycardia,
   - coronary disease,
   - non-rheumatic heart valve disease,
   - dilated cardiomyopathy of ischemic origin,
   - catheter ablation of atrial fibrillation or flutter,
   - supraventricular tachycardia other than atrial fibrillation or flutter,
   - history of valve surgery,
   - non-ischemic dilated cardiomyopathy,
   - hypertrophic cardiomyopathy,
   - rheumatic valve disease,
   - sustained ventricular tachycardia,
   - congenital cardiopathy,
   - catheter ablation for tachycardia other than for atrial fibrillation or flutter,
   - ventricular fibrillation,

and/or at least one cardiac device chosen from:

- a cardiac stimulator,
- an implantable defibrillator ("ICD").

13. Use according to anyone of the preceding claims, **characterized in that** the dose of dronedarone administered per day, orally, may reach 800 mg, taken in one or more intakes.

14. Pharmaceutical composition with an effective dose of dronedarone or of a pharmaceutically acceptable salt thereof, and also at least one pharmaceutically acceptable excipient for use in the prevention of mortality and/or of cardiovascular hospitalizations in patients having first recurrence of atrial fibrillation or atrial flutter.

FIGURE 1

FIGURE 2

FIGURE 3

Dronedarone patients with at least 1 night
of CV hospitalization (n = 195)

Placebo patients with at least 1 night
of CV hospitalization (n = 288)

☐ In ICU
▨ In medium care unit
▨ In ward

ICU, intensive care unit.

9%

18%

73%

19%

17%

64%

Europäisches
Patentamt

European
Patent Office

Office européen
des brevets

# EUROPEAN SEARCH REPORT

Application Number

EP 10 30 5504

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | Anonymous: "Assessment report for multaq"[Online] 16 December 2009 (2009-12-16), pages 1-46, XP002603885 EMEA Retrieved from the Internet: URL:http://www.ema.europa.eu/docs/en_GB/do cument_library/EPAR_-_Public_assessment_re port/human/001043/WC500044538.pdf> [retrieved on 2010-10-05] ATHENA study * pages 29-34 * * page 33, paragraph 4 and 6 * | 1-14 | INV. A61K31/343 A61P9/00 |
| X | HOHNLOSER STEFAN H ET AL: "Effect of dronedarone on cardiovascular events in atrial fibrillation." THE NEW ENGLAND JOURNAL OF MEDICINE 12 FEB 2009 LNKD- PUBMED:19213680, vol. 360, no. 7, 12 February 2009 (2009-02-12), pages 668-678, XP002603886 ISSN: 1533-4406 * page 675 * * abstract * | 1-14 | |
| X | WO 2009/144550 A2 (SANOFI AVENTIS [FR]; GAUDIN CHRISTOPHE [FR]; HAMDANI NACERA [FR]; RADZ) 3 December 2009 (2009-12-03) * claims 1-24 * * examples III.1-III.4 * | 1-14 | TECHNICAL FIELDS SEARCHED (IPC) A61K |

-/--

The present search report has been drawn up for all claims

| Place of search | Date of completion of the search | Examiner |
|---|---|---|
| Munich | 7 October 2010 | Renard, Delphine |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

**EUROPEAN SEARCH REPORT**

Application Number

EP 10 30 5504

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | STEFAN H HOHNLOSER ET AL: "Rationale and Design of ATHENA: A Placebo-Controlled, Double-Blind, Parallel Arm Trial to Assess the Efficacy of Dronedarone 400 mg Bid for the Prevention of Cardiovascular Hospitalization or Death from Any Cause in PatiENts with Atrial Fibrillation/Atrial Flutter"<br>JOURNAL OF CARDIOVASCULAR ELECTROPHYSIOLOGY, FUTURA PUBLISHING CO., ARMONK, NY, US LNKD-DOI:10.1111/J.1540-8167.2007.01016.X,<br>vol. 19, no. 1,<br>1 January 2008 (2008-01-01), pages 69-73, XP007905687<br>ISSN: 1045-3873<br>* the whole document *<br>----- | 1-14 | |
| | | | **TECHNICAL FIELDS SEARCHED (IPC)** |

The present search report has been drawn up for all claims

| Place of search | Date of completion of the search | Examiner |
|---|---|---|
| Munich | 7 October 2010 | Renard, Delphine |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 10 30 5504

This annex lists the patent family members relating to the patent documents cited in the above-mentioned European search report.
The members are as contained in the European Patent Office EDP file on
The European Patent Office is in no way liable for these particulars which are merely given for the purpose of information.

07-10-2010

| Patent document cited in search report | | Publication date | Patent family member(s) | | Publication date |
|---|---|---|---|---|---|
| WO 2009144550 | A2 | 03-12-2009 | PE | 18092009 A1 | 03-12-2009 |
| | | | US | 2010048694 A1 | 25-02-2010 |

For more details about this annex : see Official Journal of the European Patent Office, No. 12/82

# EP 2 386 300 A1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 0471609 B1

### Non-patent literature cited in the description

- **WIJFFELS MC ; KIRCHHOF CJ ; DORLAND R ; ALLESSIE MA.** Atrial fibrillation begets atrial fibrillation. A study in awake chronically instrumented goats. *Circulation,* 01 October 1995, vol. 92 (7), 1954-68

- **HOHNLOSER et al.** *Journal of cardiovascular electrophysiology,* January 2008, vol. 19 (1), 69-73